# FASCICULE DE BREVET EUROPEEN

(11) **EP 3 416 574 B1**
(45) Date de publication et mention de la délivrance du brevet: **09.09.2020**
(21) Numéro de dépôt: 17710325.6
(22) Date de dépôt: 15.02.2017
(51) Int. Cl.: A61B 17/70, A61B 17/88

(54) **IMPLANT INTERVERTÉBRAL DE STABILISATION DYNAMIQUE ET SON OUTIL DE POSITIONNEMENT**
DYNAMISCH STABILISIERENDES ZWISCHENWIRBELIMPLANTAT UND WERKZEUG ZU DESSEN POSITIONIERUNG
DYNAMICALLY STABILIZING INTERVERTEBRAL IMPLANT AND TOOL FOR POSITIONING SAME

(30) Priorité: 15.02.2016 FR 1651206
(43) Date de publication de la demande: 26.12.2018
(73) Titulaire: Backbone, 33110 Le Bouscat (FR)
(72) Inventeur: SENEGAS, Jacques, 33700 Mérignac (FR)
(74) Mandataire: Plasseraud IP
(86) Numéro de dépôt international: PCT/FR2017/050345
(87) Numéro de publication internationale: WO 2017/140983

(56) Documents cités:
- EP-A1- 2 138 122
- EP-A1- 2 184 023
- EP-A1- 2 515 778
- US-A1- 2009 292 317

## Description

### Domaine Technique

La présente invention se rapporte de manière générale aux implants utilisés dans la chirurgie du rachis, et plus particulièrement à un kit chirurgical comprenant un implant intervertébral de stabilisation dynamique ainsi que son outil de positionnement.

### Arrière-plan Technologique

Les opérations dans le domaine de la chirurgie du rachis peuvent concerner la région cervicale (nuque), la région dorsale ou, plus fréquemment, la région lombaire.

Lorsqu'il existe une instabilité, comme par exemple un glissement d'une vertèbre par rapport aux vertèbres adjacentes, une opération de stabilisation de la colonne vertébrale peut consister à implanter du matériel métallique sous forme de vis reliées entre elles par des barres ou des plaques. Ces implants constituent un échafaudage qui joue un rôle de stabilisateur de la colonne vertébrale.

Selon des techniques plus récentes, on peut obtenir la stabilisation de la colonne grâce à un implant intervertébral composé d'une cale de stabilisation, d'un lien souple de type tresse textile, d'un ensemble mobile et d'un organe de verrouillage. La cale de stabilisation est destinée à prendre place entre les apophyses épineuses de deux vertèbres consécutives, c'est-à-dire adjacentes, à stabiliser. Le lien souple (par exemple une tresse textile) enserre les apophyses. L'ensemble mobile est adapté pour venir en engagement avec la cale de stabilisation de manière à bloquer le lien souple en mouvement par rapport à la cale de stabilisation. Ce blocage est obtenu par pincement du lien souple entre l'ensemble mobile et la cale de stabilisation. L'organe de verrouillage (par exemple une vis) est adapté pour verrouiller l'engagement de l'ensemble mobile avec la cale de stabilisation, et ainsi le blocage final du lien souple qui en résulte directement.

### Art Antérieur

Un implant intervertébral du genre précité est divulgué, par exemple, dans le document EP 2515778 publié par l'OMPI sous le numéro WO 2011/077101. Plus particulièrement, ce document décrit un dispositif qui comporte un corps de cale présentant une première face et une seconde face, deux bloqueurs ou verrous positionnés sur chacune des faces et deux bandes formant liens flexibles. Le corps de cale est doté d'un orifice taraudé, prévu sur une face du corps de cale tournée vers l'arrière, qui est adapté pour recevoir la fixation d'un porte-implant. Selon l'enseignement divulgué, le porte-implant est utilisé pour la préhension de la cale lors de son positionnement dans la zone à traiter et pour le serrage des liens flexibles. En effet, un guide de serrage peut être placé par-dessus le porte-implant, pour recevoir un troisième outil servant à tendre les liens flexibles.

La plupart des opérations lombaires sont effectuées en foyer ouvert par l'arrière (voie d'abord postérieure), en pratiquant une incision dans le dos du patient au niveau des vertèbres à stabiliser.

La conception des implants de l'art antérieur implique la réalisation de gestes par le chirurgien qui nécessitent de dégager une zone d'intervention assez large autour des vertèbres à stabiliser, notamment pour la mise en place du ou des liens souples dans la cale, et pour la mise en tension et le blocage de ce ou de ces liens.

Ces dernières années, les efforts de recherche et développement se sont concentrés principalement sur les moyens et mécanismes de maintien en place de l'ensemble formé par la cale de stabilisation d'une part, et le (ou les) lien(s) souple(s) d'autre part. Plus particulièrement, l'expérience des chirurgiens montre que les aspects suivants sont à prendre en compte pour la mise en tension et la préservation de la tension du (ou des) lien(s) souple(s) :
- mode de réalisation du serrage sur le lien souple qui peut être perpendiculaire, postérieur, tangentiel, parallèle à un axe longitudinal du lien ;
- nombres de composants fixes ou mobiles nécessaires ; et,
- degré de facilité de la mise en œuvre opératoire par le chirurgien utilisateur (apprécié notamment en fonction du nombre d'étapes et du nombre d'instruments, de la position dans la plaie chirurgicale, etc.).

Le document EP 2138122 décrit un système de stabilisation entre le sacrum et une vertèbre lombaire. Le système comprend au moins une tresse textile qui est ancrée sur le sacrum par des vis. Pour assurer le blocage en tension des tresses un bloqueur comprend des pions coinceurs, entre les parois internes d'un orifice prévu dans le corps du bloqueur. La direction de déplacement d'un tel pion mobile est tangente à la surface de la tresse et est parallèle à l'axe longitudinal de ladite tresse. Ces dispositions ne sont pas adaptées à un implant intervertébral comme celui qui fait l'objet de la présente invention.

### Résumé de l'Invention

Il importe de procurer au chirurgien un implant intervertébral de stabilisation dynamique et des instruments chirurgicaux pour sa mise en place dans le contexte opératoire, qui permettent de réduire la taille de l'incision au strict minimum. En effet, il convient de protéger les tissus environnants (notamment les tissus musculaires qui participent à la stabilité de la colonne vertébrale) du stress lié à l'écartement de la plaie chirurgicale qui peut entrainer des nécroses sévères. La récupération du patient suite à l'opération chirurgicale en est d'autant plus rapide, et avec un résultat d'autant plus satisfaisant.

A cet effet, l'invention propose un Kit chirurgical comprenant :
- un implant intervertébral avec :
- une cale de stabilisation adaptée pour stabiliser entre elles au moins deux vertèbres adjacentes par interposition entre des apophyses épineuses des vertèbres, ayant un corps sensiblement parallélépipédique qui présente un axe principal déterminé et dans lequel est prévu un évidement qui présente un axe longitudinal déterminé parallèle à l'axe principal du corps de la cale de stabilisation ; et,
- au moins une sangle formant lien souple pour la fixation de la cale de stabilisation aux apophyses épineuses des vertèbres à stabiliser, ladite sangle comportant une première et une seconde portions comprenant chacune l'une des extrémités opposées de ladite sangle ; ainsi que
- un porte-implant ayant un corps allongé suivant un axe longitudinal déterminé et avec une première extrémité et une seconde extrémité, et ayant des moyens de fixation prévus à la première extrémité qui sont adaptés pour coopérer avec des moyens associés prévus au niveau du corps de la cale de stabilisation de l'implant pour la fixation du porte-implant au corps de la cale de stabilisation de telle manière que le corps allongé dudit porte implant soit en parallaxe avec ledit évidement.

Selon des modes de réalisation du kit selon l'invention :
- l'évidement prévu dans le corps de la cale de stabilisation comprend une paroi interne s'étendant parallèlement à l'axe longitudinal de l'évidement, et possède une forme déterminée ;
- au moins la première portion de sangle peut passer dans l'évidement perpendiculairement à l'axe longitudinal dudit évidement ;
- l'implant intervertébral comprend en outre un pion de blocage ayant un axe longitudinal déterminé et une forme déterminée sensiblement complémentaire de la forme de l'évidement prévu dans le corps de la cale de stabilisation, pour venir en engagement avec ladite cale de stabilisation par déplacement dans ledit évidement suivant la direction de l'axe longitudinal dudit évidement, de manière à bloquer la sangle en mouvement par rapport à la cale de stabilisation par pincement de la portion de sangle entre le pion de blocage et la paroi interne de l'évidement ; et, en outre
- le corps allongé du porte-implant est de forme tubulaire avec un canal interne s'étendant suivant l'axe longitudinal du corps allongé du porte-implant et ayant un diamètre interne légèrement plus grand que le plus grand diamètre du pion de blocage de l'implant, pour permettre l'insertion et le guidage dudit pion de blocage à travers ledit canal interne jusque dans l'évidement prévu dans le corps de la cale de stabilisation, suivant la direction de l'axe longitudinal dudit évidement, lorsque le porte-implant est fixé à la cale de stabilisation.

L'axe principal ci-dessus coïncide avec l'axe de la voie d'abord postérieure pour l'opération chirurgicale consistant à mettre l'implant en place. L'évidement prévu dans la cale de stabilisation étant ouvert postérieurement lorsque la cale est mise en position d'installation, son axe longitudinal correspond à l'axe de la voie d'abord postérieure (par opposition, par exemple, à un accès latéral, ces termes « postérieure » ou « latérale » étant ceux du vocabulaire utilisé dans ce domaine de la chirurgie du rachis). Le pion de blocage peut ainsi commodément et utilement être inséré en force dans le logement suivant l'axe de la voie d'abord postérieure. La zone d'intervention et d'insertion (foyer ouvert) peut donc être réduite au strict minimum. L'invention autorise ainsi un mode opératoire réellement mini-invasif et un traitement chirurgical court de type ambulatoire.

Si un pion tronconique mâle a déjà été utilisé pour bloquer une tresse textile dans un bloqueur tel que décrit dans le document dans EP 2138122, son utilisation telle que définie ci-dessus dans le contexte d'une cale de stabilisation destinée à prendre place entre les apophyses épineuses de deux vertèbres consécutives à stabiliser est originale. L'invention se distingue notamment au niveau de la mise en position de l'élément mobile constitué par le pion de blocage, qui est obtenue par application d'un effort suivant l'axe de la voie d'abord postérieure seulement, au moyen d'un instrument unique d'alignement, de guidage et d'insertion du pion de blocage. L'invention est également originale dans l'utilisation d'un pion de blocage en combinaison avec un lien flexible de type sangle ou tresse plate, en perpendiculaire à l'axe longitudinal de la sangle et dans le plan postérieur strict de l'implant et de la plaie chirurgicale. Ces dispositions permettent un mode opératoire réellement mini-invasif, et un traitement chirurgical court de type ambulatoire.

Selon d'autres modes de réalisation, l'évidement prévu dans le corps de la cale de stabilisation peut présenter une zone d'entrée taraudée, et le corps tubulaire du porte-implant peut présenter à sa première extrémité un filet adapté pour coopérer avec le taraudage de la zone d'entrée de l'évidement pour la fixation par vissage du porte-implant à la cale de stabilisation. Le canal interne du porte implant est ainsi directement en parallaxe avec l'évidement prévu dans la cale de stabilisation pour recevoir le pion de blocage.

Selon d'autres modes de réalisation, pris isolément ou en combinaison :
- le kit chirurgical peut comprendre, en outre, une tige d'insertion du pion de blocage ayant une première extrémité et une seconde extrémité, adaptée pour coulisser dans le canal interne du corps tubulaire du porte-implant pour l'insertion et le guidage du pion de blocage à travers le canal interne du porte-implant jusque dans l'évidement prévu dans le corps de la cale de stabilisation ; l'engagement du pion de blocage dans l'évidement est ainsi grandement facilité ;
- le kit chirurgical peut comprendre, en outre, une pièce de liaison pour fixer le pion de blocage de manière détachable à la seconde extrémité de la tige d'insertion ; on évite ainsi tout risque de chute du pion dans la plaie, lors de l'opération ; cette liaison permet aussi de manipuler le pion (par exemple en rotation autour de son axe longitudinal) directement via la tige d'insertion ;
- le pion de blocage peut comporter, à l'arrière, un évidement s'étendant suivant la direction longitudinale dudit pion ; la seconde extrémité de la tige d'insertion peut alors comprendre un évidement taraudé s'étendant suivant la direction longitudinale de ladite tige; et la pièce de liaison peut être un embout comportant d'une part une première partie extrémale filetée adaptée pour coopérer avec le taraudage de l'évidement prévu à la seconde extrémité de la tige d'insertion, et d'autre part une seconde partie extrémale conique adaptée pour être engagée à force dans l'évidement prévu à l'arrière du pion de blocage afin réaliser la fixation détachable dudit pion de blocage à ladite tige d'insertion. La pièce de liaison peut alors être à usage unique, tout en étant solidement attachée à l'extrémité de la tige d'insertion.

Dans un autre mode de réalisation, le porte-implant peut être muni d'un taraudage à sa seconde extrémité, du côté d'une entrée libre du canal interne lorsque le porte-implant est fixé à la cale de stabilisation, et la tige d'insertion peut être munie à sa première extrémité d'un filet pour coopérer avec ledit taraudage de manière que l'insertion puis le vissage de la tige d'insertion dans le canal interne du porte implant entraîne le pion conique à l'intérieur de l'évidement prévu dans le corps de la cale de stabilisation suivant la direction de l'axe longitudinal dudit évidement. Ceci permet de bien contrôler la progression du pion à l'intérieur de l'évidement prévu dans la cale de stabilisation.

Par ailleurs, la sangle présente une direction longitudinale déterminée et une surface de sangle déterminée, et le pion de blocage peut venir en engagement avec la cale de stabilisation par déplacement à l'intérieur de l'évidement prévu dans le corps de la cale de stabilisation de telle manière que l'axe longitudinal du pion de blocage :
- premièrement coïncide alors avec l'axe longitudinal de l'évidement ;
- deuxièmement est perpendiculaire à l'axe longitudinal de la portion de sangle à l'intérieur de l'évidement ; et,
- troisièmement est parallèle à la surface de la première portion de sangle à l'intérieur de l'évidement.

En variante, chacune des première et seconde portions de sangle peut passer dans le logement prévu dans la cale de stabilisation, de manière pour la sangle à former au moins une boucle dans un plan perpendiculaire à l'axe principal de la cale, avec au moins une ganse adaptée pour venir en engagement avec l'une des apophyses épineuses de deux vertèbres à stabiliser ; et le pion de blocage peut venir en engagement avec la cale de stabilisation suivant la direction de l'axe principal du corps de ladite cale entre chacune des première et seconde portions de sangle à l'intérieur de l'évidement, de telle manière que l'axe longitudinal du pion :
- premièrement coïncide alors avec l'axe longitudinal de l'évidement ;
- deuxièmement soit perpendiculaire à l'axe longitudinal de chacune des deux portions du sangle à l'intérieur de l'évidement; et,
- troisièmement soit parallèle à la surface de chacune des première et seconde portions de sangle à l'intérieur de l'évidement,
et de telle manière, en outre, que chacune des première et seconde portions de sangle soit bloquée en mouvement par rapport à la cale de stabilisation par pincement de ladite portion de sangle entre le pion de blocage et des portions respectives de la paroi interne de l'évidement se faisant face l'une à l'autre.

Les première et seconde portions de sangle peuvent alors passer dans le logement prévu dans le corps de la cale de stabilisation en sens opposés, de manière à se croiser dans ledit logement, et de manière pour la sangle à former une boucle dans un plan perpendiculaire à l'axe principal de la cale, avec deux ganses respectivement localisées de part et d'autre de la cale de stabilisation dans ledit plan et adaptées pour venir en engagement chacune avec l'une respective des apophyses épineuses de deux vertèbres adjacentes à stabiliser.

Enfin le kit chirurgical peut comprendre en outre une vis de même diamètre que le diamètre de la zone d'entrée de l'évidement prévu dans le corps de la cale de distribution avec un filet adapté pour coopérer avec le taraudage de ladite zone d'entrée, et avec une zone d'appui adaptée pour appuyer contre une zone de contact à l'arrière du pion de blocage pour verrouiller en position axiale l'engagement du pion de blocage dans l'évidement prévu dans la cale de stabilisation et ainsi le blocage de la sangle.

### Brève Description des Dessins

D'autres caractéristiques et avantages de l'invention apparaîtront encore à la lecture de la description qui va suivre. Celle-ci est purement illustrative et doit être lue en regard des dessins annexés sur lesquels :
- la Figure 1, est une vue éclatée en trois dimensions d'une partie de l'implant intervertébral selon des modes de réalisation;
- la Figure 2 est une vue de face, suivant l'axe de la voie d'abord postérieure, de l'implant assemblé selon des modes de réalisation ;
- la Figure 3 est une vue éclatée en trois dimensions d'une partie de l'implant intervertébral et d'un porte-implant associé, selon des modes de réalisation ;
- la Figure 4 une vue éclatée en trois dimensions d'une partie de l'implant intervertébral avec le porte porte-implant monté sur l'implant, et d'une tige d'insertion du pion de blocage selon des modes de réalisation ;
- la Figure 5A et la Figure 5B sont une vue en coupe, et une vue en coupe en trois dimensions, respectivement, de la cale de stabilisation munie de la sangle et du pion de blocage installé, selon des modes de réalisation ;
- la Figure 6A et la Figure 6B sont des vues identiques à celles des Figures 5A et 5B, respectivement, montrant en outre la vis de verrouillage engagée à l'entrée de l'évidement dans la cale ;
- la Figure 7A et la Figure 7B sont des vues identiques à celles des Figures 5A et 5B, respectivement, montrant en outre la vis de verrouillage en position de verrouillage ;
- la Figure 8 est une vue en trois dimensions d'un mode de réalisation du pion de blocage ;
- les Figure 9A, 9B et 9C montrent une vue de face, une vue de côté en coupe et une vue de dessus, respectivement, du pion de la Figure 8 ;
- la Figure 10 est une vue en trois dimensions d'une pièce de liaison réalisation une liaison détachable entre le pion de blocage et l'extrémité de la tige d'insertion de la Figure 4 ;
- la Figure 11 est une en trois dimensions de la tige d'insertion avec la pièce de liaison de la Figure 10 partiellement engagée dans un évidement à l'arrière du pion de blocage ;
- la Figure 12 correspond à la Figure 11 lorsque la pièce de liaison est totalement engagée dans l'évidement à l'arrière du pion de blocage ; le canal interne du porte-implant fixé à la cale de stabilisation, lors de la mise en place du pion de blocage ;
- la Figure 13 est une vue en coupe d'un porte implant fixé à la cale de stabilisation, avec la tige d'insertion engagée dans le canal du porte-implant, et le pion de blocage fixé à l'extrémité de la tige de liaison et partiellement engagé dans l'évidement prévu dans le corps de la cale de stabilisation ; et,
- la Figure 14 est une vue en trois dimensions de la cale de stabilisation avec le pion de blocage engagé dans l'évidement, avant mise en place de la vis de verrouillage.

### Description détaillée de modes de réalisation

L'implant intervertébral est destiné à prendre place entre les apophyses épineuses de deux vertèbres adjacentes, c'est-à-dire des vertèbres consécutives dans l'empilement des vertèbres lombaires, dorsales et cervicales.

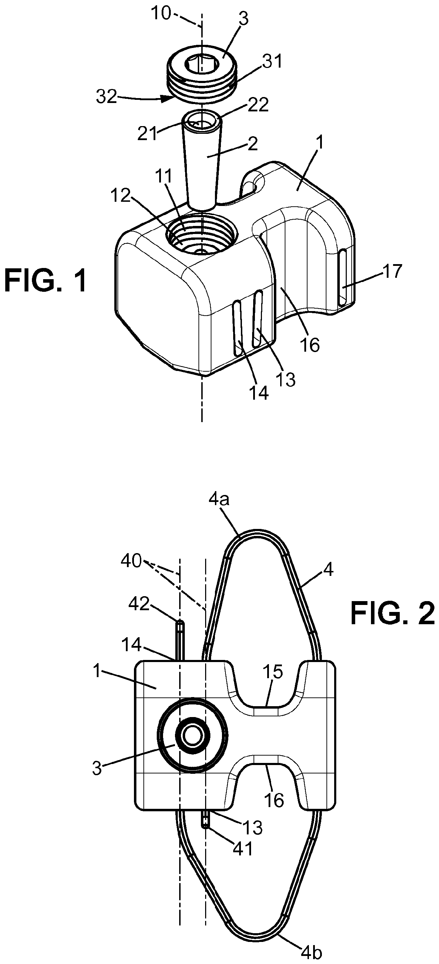

Les principaux éléments de l'implant intervertébral de stabilisation dynamique selon des modes de réalisation de la présente invention vont être décrits, tout d'abord, en référence aux Figures 1 et 2. Ainsi qu'il est montré sur ces figures, l'implant est composé d'une cale de stabilisation 1, d'un pion de blocage 2, d'une vis de verrouillage 3, et d'un lien souple 4.

La cale de stabilisation 1 comprend un corps globalement parallélépipédique avec un axe principal qui, pour des raisons de clarté de la Figure 1, est confondu sur cette figure axe l'axe longitudinal 10 d'un évidement 12 prévu dans le corps et sur lequel on reviendra plus loin. La Figure 2 est une vue de l'implant suivant l'axe longitudinal 10, lorsque l'implant est posé à plat contre les vertèbres du patient (lequel est allongé sur la table d'opération, sur le ventre). L'axe principal 10 coïncide alors avec l'axe de la voie d'abord postérieure, c'est-à-dire qu'il est perpendiculaire au dos du patient et donc à l'axe de la colonne vertébrale correspondant à la direction d'empilement des vertèbres depuis les vertèbres lombaires jusqu'aux vertèbres cervicales.

Pour ce qui est du vocabulaire descriptif, on considère dans la suite la direction d'observation du site d'implantation par le chirurgien suivant l'axe de la voie d'abord postérieure, lors de l'opération d'implantation et alors que le patient est allongé sur le ventre contre la table d'opération. Ainsi, la Figure 2 représente une vue de face suivant cet axe, et une vue de dessus suivant cette direction. Les termes « postérieurement », « avant » et « arrière », « devant » et « derrière », « avant » et « arrière », « dessus » et « dessous », « supérieur(e) » et « inférieur(e) », « haut » et « bas », « latéral(e) » et « côté », « droit(e) » et « gauche », notamment, sont utilisés dans la suite en référence à cette convention. Ces termes correspondent aussi au vocabulaire utilisé par les personnes de métier dans le domaine de la chirurgie du rachis.

Le corps de la cale de stabilisation 1 comprend, d'un côté latéral du parallélépipède, plus particulièrement à droite sur les Figures 1 et 2, une échancrure ou encoche supérieure 15 et une échancrure ou encoche inférieure 16. Ces échancrures sont adaptées pour venir en appui contre deux vertèbres à stabiliser, et plus particulièrement sur l'apophyse de la vertèbre du dessus par l'encoche 15 et sur l'apophyse de la vertèbre du dessous par l'encoche 16, respectivement. Dit autrement, dans la position installée de l'implant pour assurer la stabilisation de deux vertèbres adjacentes, les apophyses épineuses de ces vertèbres se logent dans les échancrures 15 et 16 du corps de la cale 1.

Ainsi qu'il est visible sur la Figure 1, le parallélépipède du corps de la cale 1 présente des angles adoucis (i.e. arrondis), au moins sur la face arrière destinée à être recouverte par les chairs et la peau du dos du patient. Ceci limite les risques d'inflammation ou d'endommagement des chairs du dos en contact avec l'implant. Egalement, cela réduit la gêne voire la douleur que peut ressentir le patient en cas d'appui sur cette partie de son dos, par exemple lorsqu'il est adossé contre un support (par exemple un dossier de siège) ou est allongé sur le dos (par exemple sur une surface dure comme le sol).

Le pion de blocage 2 peut avoir une forme cylindrique et conique, c'est-à-dire avoir la forme d'un cylindre dont le diamètre de la section (circulaire) se réduit progressivement suivant son axe longitudinal. Sur la Figure 1, toujours pour des raisons de clarté du dessin, l'axe longitudinal du pion coïncide avec l'axe principal 10 du corps 1 de la cale et avec l'axe longitudinal de l'évidement 12.

Le pion 2 est adapté pour coopérer avec l'évidement 12 prévu dans le corps de la cale 1, par exemple du côté latéral du parallélépipède opposé à celui où se trouvent les échancrures 15 et 16 (i.e., à gauche sur les Figures 1 et 2). A cet effet, le pion 2 et l'évidement 12 ont des formes complémentaires l'une de l'autre. Dans l'exemple représenté, le pion a la forme d'un cylindre conique, et l'évidement 12 a également une forme de cylindre conique en creux avec un angle d'ouverture égal à celui du pion et un diamètre d'ouverture légèrement plus grand que ledit celui du pion.

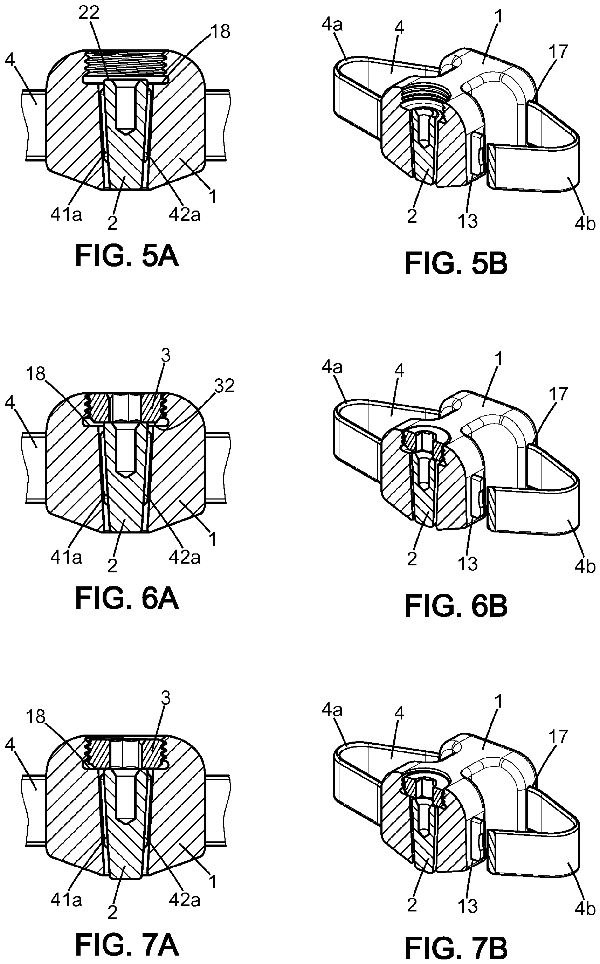

L'évidement est débouchant (ouvert) au moins du côté de la face arrière de la cale 1, et de préférence du côté de chacune des faces avant et arrière de la cale, ainsi qu'il est montré sur les Figures 5A-5B, 6A-6B, et 7A-7B. Le diamètre de l'ouverture est sensiblement plus grand que le plus grand diamètre du pion, et est constant dans une zone d'entrée de l'évidement du côté de la face arrière, par laquelle le pion 2 est destiné à entrer par son extrémité effilée. Dans cette zone d'entrée de l'évidement, ses parois présentent un filetage intérieur (taraudage) 11 pour la vis de verrouillage sur lequel on reviendra plus loin. La longueur de la zone d'entrée, suivant l'axe longitudinal, est au moins égale à l'épaisseur de la vis de verrouillage 3. En avant de cette zone d'entrée, la forme de l'évidement est conique, sans taraudage (parois lisses) et correspond sensiblement à la forme complémentaire du pion, c'est-à-dire que la forme est conique avec le même angle d'ouverture que celui du pion mais avec un diamètre légèrement plus grand pour pouvoir recevoir le pion et la tresse comme il sera explicité plus loin. Dit autrement, en avant de la zone d'entrée de l'évidement qui est taraudée, le diamètre de l'évidement décroit progressivement suivant son axe longitudinal 10 vers l'avant. Notamment, la longueur de la partie conique de l'évidement suivant l'axe longitudinal de l'évidement est sensiblement égale à la longueur du pion de blocage 2 suivant sa direction longitudinale.

La vis de verrouillage 3 est également représentée à la Figure 1 en parallaxe avec l'axe principal 10 du corps 1 de la cale et l'axe longitudinal de l'évidement 12. La vis 3 a un diamètre plus grand que le plus grand diamètre du pion. Ce diamètre correspond à celui de la zone d'ouverture taraudée de l'évidement 12. La vis 3 présente un filetage extérieur (filet) 31, adapté pour coopérer avec le taraudage 11 de l'évidement 12.

La vis 3 présente en outre une zone d'appui 32, tournée vers le bas sur la Figure 1, pouvant venir en appui contre une zone de contact 22 à l'arrière du pion 2. Dans un mode de réalisation la zone d'appui 32 de la vis 3 et/ou la zone de contact 22 du pion 2 sont des surfaces planes circulaires.

La fonction de la vis 3 est de verrouiller la position du pion 2 engagé dans l'évidement 12. Une autre fonction de la vis est, selon des modes de réalisation, de régler la position axiale du pion dans l'évidement : en tournant la vis 3 engagée dans l'évidement 12 via leurs filetages respectifs, la vis appuie par sa surface 32 contre une surface de contact 22 du côté arrière du pion 2, en sorte que celui progresse dans l'évidement 12 suivant son axe longitudinal, depuis la face arrière de la cale vers sa face avant. Des détails sur ce réglage en position seront donnés plus loin en référence aux Figures 5A-5B, 6A-6B, et 7A-7B.

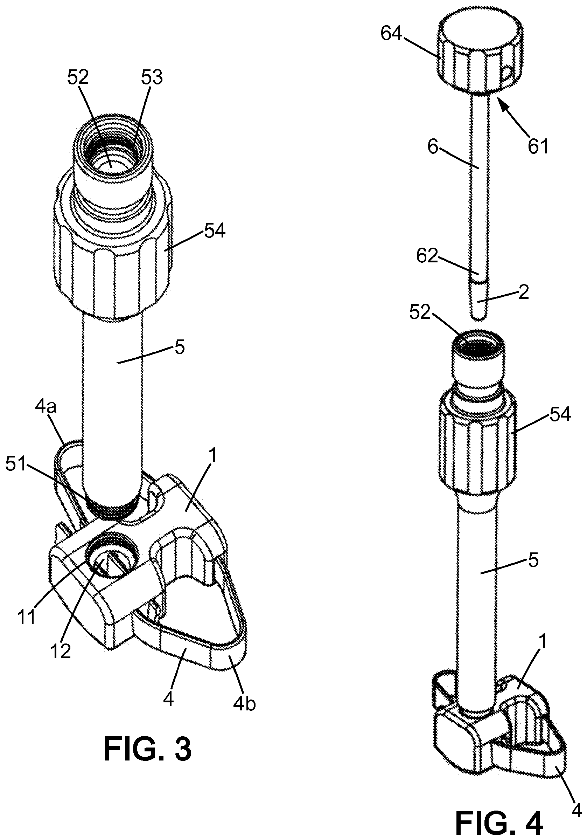

Le lien souple 4 peut être une tresse réalisée dans un matériau textile à usage médical (non résorbable), par exemple en Polyéthylène téréphtalate (PET) ou en Polyéthylène (PE). Ces matériaux peuvent être choisis en raison de leur biocompatibilité et de leur grande inertie chimique. En position installée de l'implant, le lien souple 4 enserre les apophyses d'une manière similaire à celle décrite dans le document EP 2192863, en particulier comme le montrent les Figures 3, 10 et 11A de la publication de ce document.

Le lien souple 4 a de préférence la forme d'une sangle (i.e., d'un ruban), avec un axe longitudinal et une surface de sangle s'étendant suivant ladite direction longitudinale. Il peut passer dans un passage 17 du corps de la cale prévu dans l'extrémité caudale du corps de la cale du côté droit de celle-ci, c'est-à-dire du côté des échancrures 15 et 16 qui est opposé à l'évidement 12. Le passage 17 traverse le corps de la cale 1 perpendiculairement à l'axe principal 10 du corps 1 de la cale. Lorsqu'il n'est pas bloqué en mouvement par rapport à la cale, le lien souple 4 peut coulisser dans le passage 17.

Le lien souple lien souple 4 peut également passer dans l'évidement 12. A cet effet, le corps de la cale peut présenter deux autres passages 13 et 14 traversant le corps 1 de part en part perpendiculairement à l'axe principal 10 du corps 1 de la cale. L'un au moins des passages 13 et 14, et de préférence les deux passages 13 et 14, passent par l'évidement 12. Dit autrement, les passages 13 et 14 débouchent dans l'évidement. Dans le mode de réalisation représenté, les deux passages 13 et 14 passent ainsi par l'évidement 12, mais cela n'est pas obligatoire, et il serait possible qu'un seul des passages 13 et 14 seulement traverse l'évidement 12. Lorsqu'il n'est pas bloqué en mouvement par rapport à la cale 1, le lien souple 4 peut coulisser dans les passages 13 et 14.

Le lien souple lien souple 4 est inséré manuellement par le chirurgien, par exemple d'abord au travers du passage 17. Puis les deux extrémités 41 et 42 du lien souple 4 sont tour à tour insérées dans les passages 13 et 14 après les avoir engagées par-dessus et par-dessous les apophyses épineuses des vertèbres supérieure et inférieure, respectivement.

Plus particulièrement, la sangle 4 forme alors une boucle dans un plan perpendiculaire à l'axe principal 10 de la cale 1, avec au moins une et de préférence deux ganses 4a et 4b respectivement localisées de part et d'autre de la cale dans ledit plan. Ces ganses 4a et 4b de la tresse textile sont adaptées pour venir en engagement chacune avec l'une respective des apophyses épineuses des deux vertèbres à stabiliser.

Le pion de blocage 2 peut venir en engagement avec la cale de stabilisation suivant la direction de l'axe principal 10 du corps 1 de la cale entre chacune des portions du de sangle à l'intérieur de l'évidement, de telle manière que l'axe longitudinal du pion :
- premièrement coïncide alors avec l'axe longitudinal 10 de l'évidement;
- deuxièmement soit perpendiculaire à l'axe longitudinal 40 de chacune des deux portions du sangle à l'intérieur de l'évidement; et,
- troisièmement soit parallèle à la surface de chacune des deux portions de sangle à l'intérieur de l'évidement (i.e., des portions de la sangle du côté des extrémités respectivement 41 et 42, de la sangle).

En outre, chacune des portions de la sangle du côté de ses extrémités respectivement 41 et 42, est alors bloquée en mouvement par rapport à la cale 1. Ce blocage est obtenu par pincement desdites portions de sangle entre le pion et des portions respectives de la paroi interne de l'évidement 12 se faisant face l'une à l'autre.

Pour illustrer ce blocage des deux portions de la sangle 4 par le pion 2, la partie centrale des Figures 5A, 6A et 7A correspondant à l'espace interne de l'évidement (qui reçoit le pion 2) est représentée suivant un plan de coupe à 90 degrés par rapport au plan de coupe du corps 1 de la cale sur ces mêmes figures. Ainsi, ces Figures 5A, 6A et 7A montrent en leur centre une vue en coupe des portions 41a et 42a de la sangle 40 du côté des extrémités 41 et 42 de ladite sangle, respectivement.

Dans un mode de réalisation, ces deux portions extrémales de la sangle 41a et 42a peuvent passer dans le logement 12 prévu dans la cale 1 en sens opposés, de manière à se croiser dans ledit logement et à former les ganses 4a et 4b. Ce mode de réalisation est montré aux Figures. Il n'est cependant pas exclusif. En effet, les deux portions extrémales 41a et 41b de la sangle pourraient passer parallèlement l'une à l'autre dans les passages 13 et 14, et donc dans l'évidement 12, moyennant une autre forme d'engagement avec les apophyses épineuses des vertèbres à stabiliser, et moyennant également une autre forme de la cale de stabilisation. En particulier, dans un tel mode de réalisation, la sangle ne formerait qu'une ganse, en sorte que deux sangles seraient utilisées, à savoir une pour chacune des vertèbres, avec la même cale.

Pour faciliter l'opération d'insertion dans les passages 13 et 14, les extrémités 41 et 42 de la sangle peuvent être renforcées, par exemple par traitement avec une soudeuse à ultrasons, par ajout de matière, ou par un embout en titane, ou en tout autre matériau approprié, ou par tout autre moyen équivalent.

Avantageusement, les moyens de blocage et de verrouillage du lien souple 4, qui comprennent le pion 2 et la vis 3, sont mis en place par la face postérieure de la cale de stabilisation 1. Cette disposition permet au chirurgien de réduire la taille de l'incision au strict minimum, et ainsi de conserver l'intégrité des tissus organiques environnants, notamment les muscles du dos.

Après la mise en place du lien souple 4 et sa mise en tension à l'aide d'un outil approprié (opération non décrite dans le cadre de la présente description), le pion de blocage 2 est inséré dans le logement 12, entre les deux portions extrémales du lien souple 4. L'insertion du pion 2 provoque le serrage des portions du lien souple 4 entre la surface circonférentielle externe du pion 2 et la paroi interne en regard de l'évidement 12, et stabilise le lien souple 4. En raison de cette compression du lien souple 4, celui-ci ne peut plus coulisser dans les passages 13, 14 et 17. La vis de verrouillage 3 est alors engagée dans l'évidement 12, par exemple à la main (à l'aide ou non d'un porte-outil), une fois que le pion conique 2 a été entièrement inséré dans la portion taraudée 11 de l'évidement 12 comme montré aux Figures 5A et 5B. Cette opération est illustrée par les Figures 6A et 6B. La fonction de la vis 3 est de sécuriser (en position axiale) l'engagement du pion de blocage 2 dans l'évidement 12.

La méthode de mise en place du pion conique de blocage 2 fait appel à un porte implant 5 et à une tige d'insertion 6, qui sont montrés aux Figures 3 et 4. Le porte implant 5 est par exemple un tube creux, c'est-à-dire qu'il a un corps tubulaire présentant un canal interne 52. Le canal 52 est par exemple de section circulaire constante, avec un diamètre interne légèrement plus grand que le plus grand diamètre du pion de blocage 2.

A l'une des extrémités du tube 5, il peut être prévu un taraudage 53 à l'entrée du canal 52. Egalement, des moyens de préhension pouvant avoir la forme d'une collerette cannelée 54 entourant le tube 5 comme représenté, peuvent être prévus du côté de cette extrémité d'entrée du tube 5.

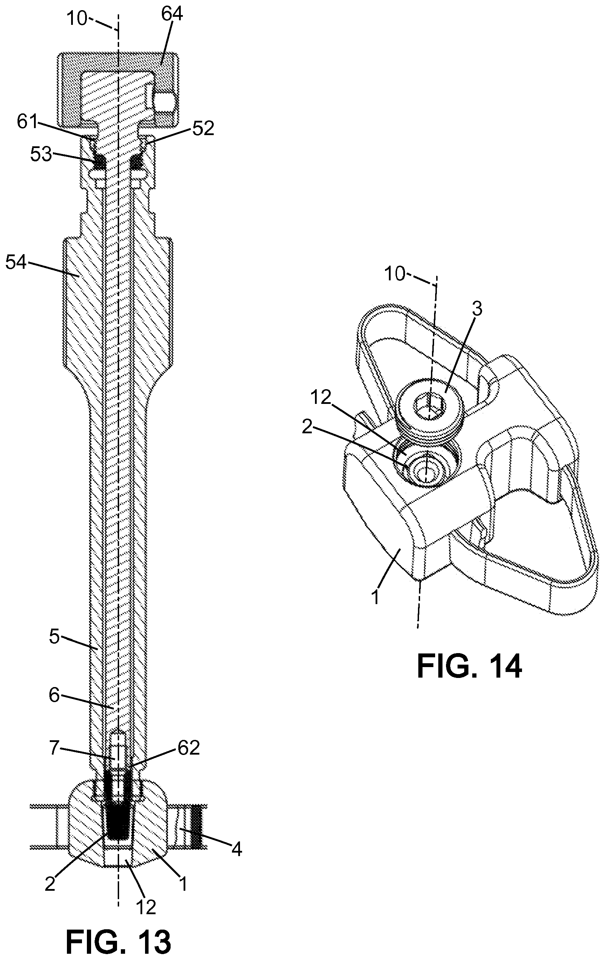

A l'autre de ses extrémités, le tube 5 peut être muni d'un filetage extérieur 51 qui coopère avec le taraudage 11 de la cale de stabilisation 1. Le porte-implant 5 peut ainsi être vissé dans la cale de stabilisation 1, avec le canal interne 52 aligné avec l'axe longitudinal 10 de l'évidement 2, c'est-à-dire en parallaxe avec l'évidement 12, en vue de l'insertion du pion de blocage 2 dans ledit évidement 12 à travers le canal interne 52. Ce mode de réalisation des moyens de fixation du porte-implant 5 au corps 1 de la cale est également illustré par la Figure 13, dont la description sera complétée plus loin.

La collerette cannelée 54 pour la préhension du porte-implant par le chirurgien est de préférence proche de l'extrémité du tube 5 correspondant à l'entrée du canal interne 52, c'est-à-dire l'extrémité opposée à celle qui porte le filet 51 par lequel le porte-implant est fixé à la cale 1.

Pour l'opération d'insertion dans l'évidement 12, le pion de blocage 2 peut être fixé de manière facilement détachable à une extrémité de la tige d'insertion 6. Cette fixation détachable peut être obtenue, par exemple, au moyen d'une pièce de liaison élastique, dont la description détaillée sera également donnée plus loin.

L'autre extrémité de la tige d'insertion 6 est munie d'un filet 61 externe en dessous de moyens de préhension tels qu'une tête cannelée 63 comme représenté. Ce filet (non visible à la Figure 4), qui coopère avec le taraudage 53 du porte-implant 5.

La tige d'insertion 6 avec le pion conique 2 maintenu de manière détachable à son extrémité, sont introduits dans le canal 52 du porte-implant 5, par son extrémité portant le taraudage 53. Le vissage de la tige d'insertion 6 dans le canal 52 du porte implant 5 entraîne le pion conique 2 dans le logement 12 de la cale 1. Ce vissage est réalisé par la coopération du filet 61 de la tige 6 et du taraudage 53 du porte-implant 5. Ainsi, on obtient l'engagement à force du pion 2 dans la portion conique et non taraudée de l'évidement 12, ainsi qu'il a été indiqué plus haut, par ce vissage de la tige d'insertion dans le porte-implant. Après vissage complet de la tige d'insertion dans le porte-implant, le pion 2 est complètement engagé dans la portion conique de l'évidement 12. Il vient alors en engagement avec la paroi interne de l'évidement en pinçant le lien souple 4 contre cette paroi. L'écrasement de la tresse qui résulte de ce pincement contre la paroi interne de l'évidement donne une certaine élasticité, très faible, à l'engagement du pion dans ledit évidement.

Le porte implant 5 peut alors être dévissé de la cale 1, éventuellement après dévissage de la tige d'insertion du porte-implant (non obligatoire). Le pion 2 se détache alors automatiquement de l'extrémité de la tige d'insertion 6 en restant en place dans la portion conique non taraudée de l'évidement 12. A cet effet, la force de la liaison du pion 2 à l'extrémité 62 de la tige d'insertion 2 est calibrée de manière à être inférieure à la pression de serrage appliquée sur le pion 2 par la paroi interne de la portion conique non taraudée de l'évidement 12 lorsque le pion y est totalement engagé, et par la force de réaction au pincement du ou des liens flexibles.

Lors de l'opération d'insertion du pion 2 dans l'évidement 12, la cale de stabilisation 1 peut être maintenue par le chirurgien, en position avec les ganses 4a et 4b de la sangle 4 engagées autour des apophyses épineuses des vertèbres à stabiliser, grâce notamment à la zone de préhension 54 du porte-implant 5.

Le porte-implant sert donc simultanément au maintien de la cale de stabilisation en position contre les vertèbres lors de l'étape critique consistant à insérer le pion de blocage du lien souple 4, mais aussi d'instrument d'alignement, de guidage et d'insertion du pion grâce à la tige 6 qui coulisse dans le canal interne 52 du porte-implant.

Une fois le porte-implant 5 désolidarisé de la cale 1 (laquelle est déjà ainsi en situation de stabilisation des vertèbres), la vis de verrouillage 3 peut être engagée dans la portion taraudée 11 de l'évidement 12 prévu dans la cale, comme montré à la Figure 6A et à la Figure 6B. On notera que, à cette étape, le lien souple est déjà bloqué par le pion 2, par pincement des portions extrémales 41a et 42a contre des portions opposées de la paroi interne de l'évidement 12, comme montré à la Figure 5A et à la Figure 5B.

La vis 3 et le pion conique 2 permettent de maîtriser la pression de serrage exercée sur le lien souple 4 sans avoir recours à un dispositif de mesure de la force ou du couple de serrage.

A cet effet, la longueur du pion conique 2 suivant sa direction longitudinale est calibrée pour que sa surface plane de contact 22 affleure très légèrement au-dessus du lamage du trou taraudé 11 de la cale de stabilisation 1 lorsqu'il est inséré selon la technique décrite précédemment. La face d'appui plane 32 de la vis 3 vient alors en appui sur le lamage du trou taraudé 11, ainsi qu'en appui sur la surface plane de contact 22 du pion conique 2, du côté arrière, comme montré à la Figure 7A et à la Figure 7B.

Le vissage de la vis 3 dans le trou taraudé 11 s'effectue par exemple à la main, à l'aide d'un tournevis, par exemple à tête hexagonale. Lorsque la surface plane 32 de la vis de serrage entre en contact avec la surface de contact 22 du verrou de blocage, la vis de serrage entraîne le pion de blocage 2 plus en avant dans l'évidement 12, jusqu'à ce que la surface plane 32 de la vis de serrage rencontre le lamage du trou taraudé 11 qui forme un épaulement. La progression axiale du pion conique 2 est alors bloquée par cet épaulement, assurant que la pression exercée par le pion conique 2 sur le lien souple 4 n'augmente plus même si le chirurgien continue d'exercer un couple de serrage sur la vis de serrage 3 à l'aide du tournevis.

Dans cette position, la vis 3 empêche tout mouvement du pion 2 qui tendrait à le faire ressortir de l'évidement 12, par exemple sous l'effet de tensions exercées sur le lien souple 4 par les mouvements du patient dans les gestes de la vie courante. C'est la fonction de verrouillage de la vis 3.

L'originalité de la fonction du pion de blocage selon des modes de réalisation de l'invention réside dans la direction de déplacement du pion qui est orthogonale à la direction de la force d'appui du pion contre la surface de la sangle 4 et contre la paroi interne de l'évidement 12 prévu dans la cale. Le pion se déplace en effet uniquement suivant l'axe de la voie d'abord postérieure, ce qui limite l'étendue de la zone d'intervention chirurgicale.

La solution décrite dans la présente description permet en outre d'installer une vis de verrouillage 3 qui procure une sécurité (verrouillage en position) du pion de blocage 2 de la sangle 4, et permet un contrôle de la pression exercée en raison de la maîtrise du déplacement axial du pion conique 2 dans l'évidement 12. Cette vis est également mise en place et serrée uniquement suivant la direction de l'axe de la voie d'abord postérieure.

En d'autres termes, et suivant le principal avantage des modes de réalisations décrits, aucune action du chirurgien ni aucun mouvement de pièce mobile n'est effectué suivant une direction latérale. Tout se passe suivant l'axe de la voie d'abord postérieure.

La cale de stabilisation 1 peut être réalisée en polymère, par exemple en polyéther éther cétone (PEEK). Elle peut être obtenue par usinage à partir d'une barre ou d'un bloc de matériau brut, par injection moulée, par impression 3D, ou par toute autre technique équivalente.

Le pion de blocage conique 2 est de préférence réalisé en alliage de titane, choisi pour sa résistance mécanique et sa biocompatibilité. Il peut par exemple être obtenu par usinage à partir d'une barre de matériau brut.

La vis 3 de serrage et de blocage verrouillage peut également être en alliage de titane, pour les mêmes raisons et avec les mêmes avantages. Elle peut être obtenue par usinage à partir d'une barre de matériau brut.

Le lien souple 4 est fabriqué, de préférence, en textile tressé. Ainsi qu'il a déjà été dit plus haut, les extrémités du lien souple peuvent être rigidifiées de manière à faciliter leur préhension et leur guidage au travers des passages 13, 14 et 17 prévus dans le corps de la cale de stabilisation 1.

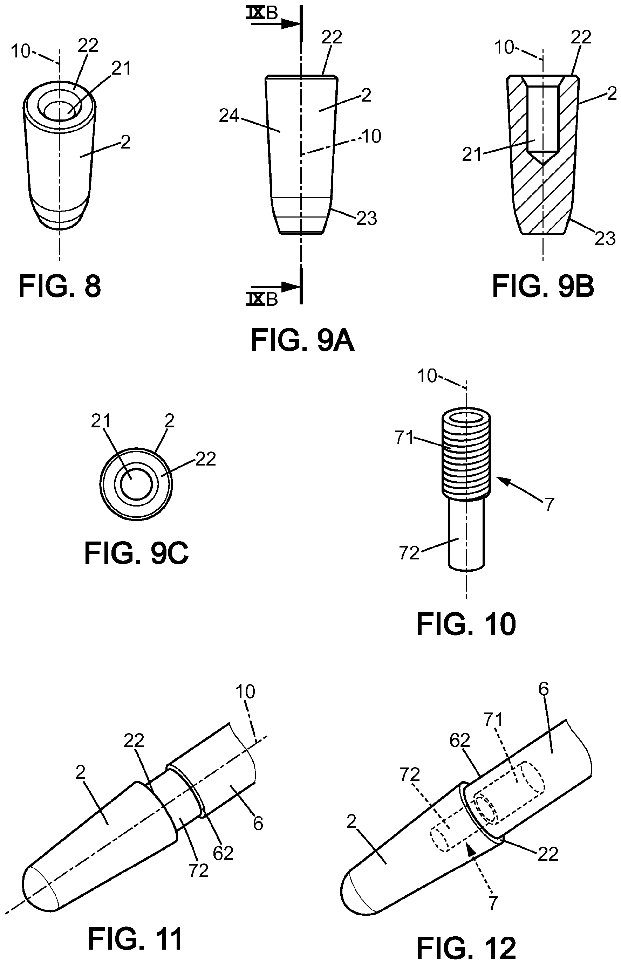

En référence à la Figure 8 et aux Figures 9A, 9B et 9C, le pion présente une forme générale tronconique. Du côté le plus effilé, dit côté « avant » c'est-à-dire celui par lequel le pion est inséré dans l'évidement suivant l'axe de la voie d'abord postérieure, et c'est-à-dire aussi celui qui est opposé au côté dit « arrière » qui est muni de la surface d'appui 22 déjà présentée, le pion est muni d'une extrémité 23 arrondie. Cette extrémité arrondie facilite son passage entre les portions de sangle de la sangle 4 lorsqu'elles sont mises en tension dans l'évidement 12 prévu dans le corps 1 de la cale de stabilisation.

Dans le mode de réalisation ici représenté, le pion 2 comprend un évidement 21 s'étendant suivant son axe longitudinal 10, par exemple de diamètre égal à 2 mm, qui est débouchant à l'extrémité arrière 22 du pion. Cet évidement permet de recevoir une pièce de liaison, sur laquelle on reviendra plus loin, qui a pour fonction de fixer le pion de manière détachable à l'extrémité de la tige d'insertion 6. Mais cette liaison n'est pas obligatoire, le pion pouvant être simplement poussé à l'aide de la tige d'insertion 6, après son introduction dans le canal interne 52 du porte-implant 5, jusque dans l'évidement 12 prévu dans le corps 1 de la cale.

Le fut central du pion 4 présente un plus grand diamètre du côté de l'extrémité arrière 22, par exemple 4,4 mm, et un plus petit diamètre, par exemple 3,9 mm du côté de l'extrémité avant. Les dimensions données ci-dessus sont purement illustratives d'un mode de réalisation non limitatif donné pour les besoins de la présente description.

Comme évoqué plus haut, des moyens peuvent être prévus pour fixer le pion de manière détachable à la tige d'insertion 6, à l'extrémité 62 de celle-ci. Cette fixation n'est pas obligatoire, ainsi qu'il a été précédemment indiqué. Mais elle apporte l'avantage d'éviter la manipulation du pion seul, qui est une pièce de petite taille, au-dessus du champ opératoire. On limite ainsi le risque lié à la chute éventuelle du pion, dans le site d'implantation ou en dehors. La liaison détachable du pion à la tige permet aussi de maîtriser la progression du pion 2 suivant l'axe longitudinal de l'évidement 12 prévu dans le corps de la cale, par le vissage de la tige d'insertion comme on le verra plus loin.

La liaison du pion 2 à la tige d'insertion 6 peut être réalisée par une pièce intermédiaire de liaison 7, telle que l'embout représenté à la Figure 10.

L'embout 7 comporte ici, d'une part, une première partie extrémale 71 filetée. Cette partie 71 est adaptée pour coopérer avec le taraudage d'un évidement taraudé qui est prévu à la seconde extrémité 62 de la tige d'insertion 6 et qui s'étend suivant la direction longitudinale 10 de ladite tige 6.

L'embout 7 comporte d'autre part une seconde partie extrémale 72, qui peut être légèrement conique, sans être filetée. Cette partie 72 est adaptée pour être engagée à force dans l'évidement 21 qui est prévu à l'arrière du pion de blocage 2 et qui a été présenté ci-dessus en référence à la Figure 8 et aux Figures 8, 9B et 9C. La forme légèrement conique de la seconde partie 72 de l'embout facilite cette opération d'engagement à force.

A la Figure 11, l'embout 7 est représenté avec la première partie 71 complètement vissée dans l'évidement prévu à l'extrémité 62 de la tige s'insertion 6. En outre, la seconde partie 72 de l'embout n'est ici que partiellement engagée dans l'évidement 21 prévu à l'arrière 22 du pion 2.

Il est préférable de rapprocher le pion de la tige jusqu'à ce que l'intégralité de la partie 72 de l'embout 7 soit engagée dans l'évidement 21 du pion, c'est-à-dire jusqu'à ce que l'extrémité 62 de la tige vienne en contact avec la face arrière 22 du pion 2. Cette opération est réalisée par le chirurgien en saisissant le pion 2 dans une main et la tige 6 dans l'autre main. De petits mouvements de rotation entre le pion et la tige peuvent être exercés simultanément à l'application d'une force de rapprochement entre ces deux éléments, pour faciliter l'engagement total du pion par-dessus la partie 72 de l'embout.

La Figure 12 montre le pion 2 après achèvement complet de sa liaison à l'extrémité 62 de la tige d'insertion 6. Sur cette figure, l'embout 7 n'est plus visible, et a été représenté en fantôme (i.e., en traits pointillés) afin d'illustrer la manière dont la liaison du pion 2 et de la tige 6 est réalisée.

La partie 71 réalise la fixation détachable du pion de blocage 2 à la tige d'insertion 6. En effet, une fois que le pion 2 a été mis en place dans l'évidement 12 prévu dans la cale 1 alors qu'une tension était exercée sur le ou les liens souples 4, on relâche la force de tension qui était exercée sur les liens 4, lesquels restent alors coincés contre les parois de l'évidement 12 et conservent donc leur tension. On dévisse alors le porte-implant 5 de la cale de stabilisation 1, avec toujours la tige d'insertion 6 vissée à l'intérieur du canal interne 51 du porte-implant 5. Cela exerce une traction sur la tige d'insertion 6 suivant la direction longitudinale, vers l'arrière. La partie 71 de l'embout 7 se désengage alors du pion 2, tandis que le pion reste en engagement dans l'évidement 12 prévu dans le corps de la cale.

L'embout 7 peut être réalisé en PE, en PET ou en PEEK, ou autre, par usinage, moulage, impression 3D, ou autre. Compte tenu de la déformation qu'il peut subir lors de son insertion à force dans l'arrière du pion de blocage 2, l'embout est de préférence à usage unique. C'est notamment pour cette raison qu'il se présente sous la forme d'une pièce indépendante pouvant être vissée à l'extrémité 62 de la tige 6. Il peut en effet être remplacé après chaque intervention, alors que le porte-implant et la tige d'insertion sont réalisés en acier inoxydable et peuvent être réemployés.

De préférence, le kit est fourni au chirurgien avec l'embout 7 déjà vissé dans la tige d'insertion 6. Par ailleurs, le kit peut être livré, en outre, avec le pion 2 déjà couplé à la tige d'insertion par l'embout 7. Néanmoins, le chirurgien peut préférer assembler lui-même le pion sur la partie 72 de l'embout, afin de pouvoir apprécier la force de la liaison ainsi réalisée. Le chirurgien peut ainsi vérifier que cette liaison est bien détachable en ce sens que l'embout se désengagera facilement du pion lors du retrait du porte-implant 5 comme exposé au paragraphe précédent.

La liaison entre le pion de blocage 2 et la tige d'insertion 6 est également visible à la Figure 13 qui est une vue en coupe de la tige d'insertion 2 lorsqu'elle est introduite dans le canal interne du porte-implant 5 pour la mise en place du pion 2 dans l'évidement 11 prévu dans le corps 1 de la cale de stabilisation. On voit sur cette figure, sur laquelle les mêmes éléments qu'aux autres figures portent les mêmes références, que le porte-implant 5 est fixé à la cale de stabilisation 1 et qu'un lien souple 4 (sangle) passe dans l'évidement 12.

Dans le mode de réalisation présenté à la Figure 13, le porte-implant est muni d'un taraudage 53 à l'autre de ses extrémités, du côté d'une entrée libre du canal interne 52, c'est-à-dire le côté opposé à la cale 1 lorsque le porte-implant est fixé à ladite cale 1. Le terme «entrée» est ici utilisé en référence au fait que le pion est inséré dans le canal interne 52 du porte implant par cette extrémité taraudée 53 de la tige. La tige d'insertion 6 est munie d'un filet complémentaire 61, à son extrémité 62 pour coopérer avec le taraudage 53. De cette manière, l'insertion puis le vissage de la tige d'insertion 6 dans le canal interne 52 du porte-implant 5 fait progresser le pion conique 2 dans l'évidement 12 prévu dans le corps 1 de la cale de stabilisation, suivant la direction de l'axe longitudinal 10 dudit évidement (lequel coïncide alors avec l'axe longitudinal du canal interne 52). La progression du pion suivant cette direction est bien maîtrisée par le contrôle de la rotation de la tige d'insertion. Cette opération peut être réalisée par le chirurgien en tenant le porte-implant 5 dans une main via la partie de préhension 54 dudit porte-implant, et en tournant la tige d'insertion 6 avec l'autre main via la partie de préhension 64 de ladite tige.

On notera qu'il est avantageux que l'insertion du pion suivant l'axe longitudinal de l'évidement 12 soit réalisée par rotation du pion autour dudit axe. En effet, le mouvement hélicoïdal du pion qui est ainsi obtenu facilite son insertion dans l'évidement et limite le stress sur les liens souples qu'il vient coincer contre la paroi interne de l'évidement. Plus particulièrement, le frottement du pion 2 sur la surface des liens souples, ou surface de sangle, est réparti entre une composante orthogonale à la direction longitudinale de la sangle et une composante parallèle à ladite direction longitudinale. Cela limite également le risque que la sangle ne se replie sur elle-même suivant la direction orthogonal à l'axe longitudinale de la sangle.

Cet effet avantageux supplémentaire n'est pas obtenu si le pion 2 est simplement poussé longitudinalement par la tige d'insertion 6, c'est-à-dire sans vissage de celle-ci dans le porte-implant (par exemple si la tige coulisse dans le canal interne 52 du porte-implant sans rotation autour de son axe longitudinal 10). De même, cet effet n'est pas obtenu sans la liaison entre le pion 2 et la tige d'insertion 6.

Lorsque la tige d'insertion a été vissée à fond dans le canal interne 52 du porte-implant, le pion de blocage est complètement inséré dans l'évidement 12 prévu dans la cale de stabilisation. Ce résultat est garanti par la conception des différents éléments du kit, notamment la longueur axiale du pion et celle de la tige d'insertion, et la longueur axiale des taraudages 11 et 53.

Le porte-implant peut alors être séparé de la cale de stabilisation, en le dévissant de la portion taraudée 11 de l'évidement 12 prévu dans ladite cale, en laissant le pion 2 en place dans ledit évidement, ainsi qu'il a été dit plus haut.

La Figure 14 est une vue en trois dimensions de la cale de stabilisation avec le pion de blocage 2 engagé dans l'évidement, après séparation du porte-implant 5 de la cale de stabilisation 1, et avant mise en place de la vis de verrouillage 3. On notera à nouveau que le blocage du lien souple 4 est efficace avec le pion conique 2 seul, la vis de verrouillage 3 n'étant qu'une mesure de sécurité supplémentaire.

Avantageusement, la même partie taraudée 11 à l'entrée de l'évidement 12 dans la cale de stabilisation, sert à nouveau, cette fois-ci pour la mise en place de la vis de verrouillage 3. Dit autrement, le taraudage 11 sert successivement à la fixation temporaire du porte-implant 5 sur la cale 1 pendant l'opération, puis à la mise en place de la vis de verrouillage 3 qui sécurise à demeure la position du pion 2 dans l'évidement 12. La conception de la cale selon les modes de réalisations décrits est donc particulièrement simple. Un nombre réduit de pièces et d'éléments permettent la mise en place du pion de blocage par des opérations manuelles qui sont réalisées par le chirurgien suivant l'axe de la voie d'abord postérieure uniquement.

L'invention a été décrite et illustrée dans la présente description détaillée et dans les Figures, dans des formes de réalisation particulièrement avantageuses. Elle ne se limite pas, toutefois aux formes de réalisation présentées. D'autres variantes et modes de réalisation peuvent être déduits et mis en oeuvre par la personne du métier à la lecture de la présente description et des dessins annexés.

Notamment, la fixation du porte-implant 5 sur la cale 1 peut être réalisée d'une manière différente du vissage décrit plus haut. Par exemple, cette fixation peut être obtenue par un mécanisme de type « à baïonnette », ou par toute autre forme d'encliquetage.

En outre, la liaison entre le pion 2 et la tige d'insertion 6 peut être obtenue grâce à une pièce de liaison 7 qui peut différer, dans sa forme de réalisation, de l'embout décrit plus haut en référence à la Figure 10. La pièce 7 peut par exemple être une pièce de liaison élastique. Il peut s'agir par exemple d'un cylindre ou manchon en matériau élastique déformable, par exemple en matériau élastomère. Cette pièce 7 coopère avec l'évidement 21 réalisé à l'arrière 22 du pion de blocage 2, et avec un évidement non taraudé prévu au niveau de la première extrémité 62 de la tige d'insertion 6. Lorsqu'une première partie extrémale du manchon est enfoncée dans le premier évidement ci-dessus, et que la seconde partie extrémale du manchon est enfoncée dans le second évidement ci-dessus, le manchon réalise la fixation détachable dudit pion de blocage à la tige d'insertion.

A cet effet, le diamètre du manchon 7 est légèrement supérieur au diamètre de l'évidement 21 qui est prévu dans le pion 2. Ainsi, le manchon 7 peut être introduit à force dans le canal 21 par l'extrémité arrière 22 du pion 2, en jouant de son élasticité. Une partie du manchon est laissée en dehors du canal 21, et dépasse de l'extrémité 22 du pion vers l'arrière. Le chirurgien enfonce alors cette extrémité libre du manchon 7, dont l'autre extrémité est et demeure engagée dans le pion 2, à l'intérieur de l'évidement à l'extrémité 62 de la tige d'insertion. Le diamètre de cet évidement est légèrement inférieur au diamètre de la pièce 7. Dit autrement, la tige 6 est ainsi enfoncée par-dessus le manchon 7, et ce jusqu'à ce que le bord de l'extrémité 62 de la tige entre en contact avec le bord arrière 22 du pion de blocage 2. L'ordre des deux opérations ci-dessus peut être inversé.

Dans les revendications, les termes « comprend » ou « comporte » n'excluent pas d'autres éléments ou d'autres étapes. Les différentes caractéristiques présentées et/ou revendiquées peuvent être avantageusement combinées. Leur présence dans la description ou dans des revendications dépendantes différentes, n'excluent pas cette possibilité. Les signes de référence ne sauraient être compris comme limitant la portée de l'invention.

## Revendications

1. Kit chirurgical comprenant :
- un implant intervertébral avec :
- une cale de stabilisation (1) adaptée pour stabiliser entre elles au moins deux vertèbres adjacentes par interposition entre des apophyses épineuses des vertèbres, ayant un corps sensiblement parallélépipédique qui présente un axe principal (10) déterminé et dans lequel est prévu un évidement (12) qui présente un axe longitudinal déterminé parallèle à l'axe principal du corps de la cale de stabilisation ; et,
- au moins une sangle (4) formant lien souple pour la fixation de la cale de stabilisation aux apophyses épineuses des vertèbres à stabiliser, ladite sangle comportant une première et une seconde portions (41a,42a) comprenant chacune l'une des extrémités opposées (41,42) de ladite sangle ; ainsi que
- un porte-implant (5) ayant un corps allongé suivant un axe longitudinal déterminé et avec une première extrémité (51) et une seconde extrémité (53), et ayant des moyens de fixation prévus à la première extrémité (51) qui sont adaptés pour coopérer avec des moyens associés prévus au niveau du corps de la cale de stabilisation de l'implant pour la fixation du porte-implant au corps de la cale de stabilisation de telle manière que le corps allongé dudit porte implant soit en parallaxe avec ledit évidement (12) ;
dans lequel :
- l'évidement prévu dans le corps de la cale de stabilisation comprend une paroi interne s'étendant parallèlement à l'axe longitudinal de l'évidement, et possède une forme déterminée ;
- au moins la première portion de sangle peut passer dans l'évidement perpendiculairement à l'axe longitudinal dudit évidement ;
- l'implant intervertébral comprend en outre un pion de blocage (2) ayant un axe longitudinal déterminé et une forme déterminée sensiblement complémentaire de la forme de l'évidement prévu dans le corps de la cale de stabilisation, pour venir en engagement avec ladite cale de stabilisation par déplacement dans ledit évidement suivant la direction de l'axe longitudinal dudit évidement, de manière à bloquer la sangle en mouvement par rapport à la cale de stabilisation par pincement de la portion de sangle entre le pion de blocage et la paroi interne de l'évidement ; et,
- le corps allongé du porte-implant est de forme tubulaire avec un canal interne (52) s'étendant suivant l'axe longitudinal du corps allongé du porte-implant et ayant un diamètre interne légèrement plus grand que le plus grand diamètre du pion de blocage (2) de l'implant, pour permettre l'insertion et le guidage dudit pion de blocage à travers ledit canal interne jusque dans l'évidement (12) prévu dans le corps de la cale de stabilisation, suivant la direction de l'axe longitudinal dudit évidement, lorsque le porte-implant est fixé à la cale de stabilisation.

2. Kit chirurgical selon la revendication 1, dans lequel :
- l'évidement prévu dans le corps de la cale de stabilisation présente une zone d'entrée (11) taraudée, et
- le corps tubulaire du porte-implant présente à sa première extrémité (51) un filet adapté pour coopérer avec le taraudage de la zone d'entrée de l'évidement pour la fixation par vissage du porte-implant à la cale de stabilisation (1).

3. Kit chirurgical selon la revendication 1 ou la revendication 2, comprenant en outre :
- une tige (6) d'insertion du pion de blocage (2) ayant une première extrémité (61) et une seconde extrémité (62), adaptée pour coulisser dans le canal interne (52) du corps tubulaire du porte-implant (5) pour l'insertion et le guidage du pion de blocage (2) à travers le canal interne (52) du porte-implant (5) jusque dans l'évidement (12) prévu dans le corps de la cale de stabilisation.

4. Kit chirurgical selon la revendication 3, comprenant en outre une pièce de liaison (7) pour fixer le pion de blocage (2) de manière détachable à la seconde extrémité (62) de la tige d'insertion (6).

5. Kit chirurgical selon la revendication 4, dans lequel :
- le pion de blocage (2) comporte à l'arrière (22) un évidement (21) s'étendant suivant la direction longitudinale (10) dudit pion,
- la seconde extrémité (62) de la tige d'insertion (6) comprend un évidement taraudé s'étendant suivant la direction longitudinale de ladite tige (6), et
- la pièce de liaison (7) est un embout comportant d'une part une première partie extrémale (71) filetée adaptée pour coopérer avec le taraudage de l'évidement prévu à la seconde extrémité (62) de la tige d'insertion, et d'autre part une seconde partie extrémale (72) conique adaptée pour être engagée à force dans l'évidement prévu à l'arrière du pion de blocage (2) afin réaliser la fixation détachable dudit pion de blocage à ladite tige d'insertion.

6. Kit chirurgical selon l'une des revendications 1 à 5, dans lequel le porte-implant (5) est muni d'un taraudage à sa seconde extrémité (53), du côté d'une entrée libre du canal interne (52) lorsque le porte-implant est fixé à la cale de stabilisation, et dans lequel la tige d'insertion (6) est munie à sa première extrémité (61) d'un filet pour coopérer avec ledit taraudage de manière que l'insertion puis le vissage de la tige d'insertion (6) dans le canal interne (52) du porte implant (5) entraîne le pion conique (2) à l'intérieur de l'évidement (12) prévu dans le corps de la cale de stabilisation suivant la direction de l'axe longitudinal dudit évidement.

7. Kit chirurgical selon l'une quelconque des revendications 1 à 6, dans lequel la sangle présente une direction longitudinale déterminée et une surface de sangle déterminée, et dans lequel le pion de blocage peut venir en engagement avec la cale de stabilisation par déplacement à l'intérieur de l'évidement prévu dans le corps de la cale de stabilisation de telle manière que l'axe longitudinal du pion de blocage :
- premièrement coïncide alors avec l'axe longitudinal de l'évidement ;
- deuxièmement est perpendiculaire à l'axe longitudinal de la portion de sangle à l'intérieur de l'évidement ; et,
- troisièmement est parallèle à la surface de la première portion de sangle à l'intérieur de l'évidement.

8. Kit chirurgical selon l'une quelconque des revendications 1 à 6, dans lequel :
- la sangle présente une direction longitudinale déterminée et une surface de sangle déterminée ;
- chacune des première et seconde portions de sangle peut passer dans le logement prévu dans la cale de stabilisation, de manière pour la sangle à former au moins une boucle dans un plan perpendiculaire à l'axe principal de la cale, avec au moins une ganse adaptée pour venir en engagement avec l'une des apophyses épineuses de deux vertèbres à stabiliser ; et
- le pion de blocage peut venir en engagement avec la cale de stabilisation suivant la direction de l'axe principal du corps de ladite cale entre chacune des première et seconde portions de sangle (41a,42a) à l'intérieur de l'évidement, de telle manière que l'axe longitudinal du pion :
- premièrement coïncide alors avec l'axe longitudinal de l'évidement ;
- deuxièmement soit perpendiculaire à l'axe longitudinal de chacune des deux portions du sangle à l'intérieur de l'évidement; et,
- troisièmement soit parallèle à la surface de chacune des première et seconde portions de sangle à l'intérieur de l'évidement,
et de telle manière, en outre, que chacune des première et seconde portions de sangle soit bloquée en mouvement par rapport à la cale de stabilisation par pincement de ladite portion de sangle entre le pion de blocage et des portions respectives de la paroi interne de l'évidement se faisant face l'une à l'autre.

9. Kit chirurgical selon la revendication 8, dans lequel les première et seconde portions de sangle peuvent passer dans le logement prévu dans le corps de la cale de stabilisation en sens opposés, de manière à se croiser dans ledit logement, et de manière pour la sangle à former une boucle dans un plan perpendiculaire à l'axe principal de la cale, avec deux ganses respectivement localisées de part et d'autre de la cale de stabilisation dans ledit plan et adaptées pour venir en engagement chacune avec l'une respective des apophyses épineuses de deux vertèbres adjacentes à stabiliser.

10. Kit chirurgical selon l'une quelconque des revendications précédentes, comprenant en outre une vis de même diamètre que le diamètre de la zone d'entrée de l'évidement prévu dans le corps de la cale de stabilisation avec un filet adapté pour coopérer avec le taraudage de ladite zone d'entrée, et avec une zone d'appui (32) adaptée pour appuyer contre une zone de contact à l'arrière (22) du pion de blocage (2) pour verrouiller en position axiale l'engagement du pion de blocage dans l'évidement (12) prévu dans la cale de stabilisation et ainsi le blocage de la sangle.

## Patentansprüche

1. Chirurgie-Kit bestehend aus:
- einem Zwischenwirbel-Implantat mit:
- einem Stabilisierungskeil (1), der dazu ausgebildet ist, wenigstens zwei benachbarte Wirbel durch Einfügung zwischen Dornfortsätzen der Wirbel zu stabilisieren, mit einem im wesentlichen parallelepipedischen Körper, der eine vorbestimmte Hauptachse (10) aufweist und in dem eine Aussparung (12) vorgesehen ist, die eine bestimmte Längsachse parallel zur Hauptachse des Körpers des Stabilisierungskeils aufweist; und
- wenigstens einem Riemen (4), der eine flexible Verbindung zur Befestigung des Stabilisierungskeils an den Dornfortsätzen der zu stabilisierenden Wirbel bildet, wobei der Riemen einen ersten und einen zweiten Abschnitt (41a, 42a) umfasst, die jeweils eines der gegenüberliegenden Enden (41, 42) des Riemens aufweisen; sowie
- einem Implantathalter (5) mit einem länglichen Körper entlang einer vorbestimmten Längsachse und mit einem ersten Ende (51) und einem zweiten Ende (53), und mit an dem ersten Ende (51) vorgesehen Befestigungsmitteln, welche dazu ausgebildet sind, mit zugehörigen Mitteln, die an dem Körper des Implantatstabilisierungskeils vorgesehen sind, zusammenzuwirken, um den Implantathalter an dem Körper des Stabilisierungskeils derart zu befestigen, dass der längliche Körper des Implantathalters in Parallaxe mit der Aussparung (12) ist;
wobei :
- die im Körper des Stabilisierungskeils vorgesehene Aussparung eine Innenwand umfasst, die sich parallel zur Längsachse der Aussparung erstreckt, und eine vorbestimmte Form hat; und
- wenigstens der erste Riemenabschnitt in der Aussparung senkrecht zur Längsachse der Aussparung verlaufen kann;
- das Zwischenwirbelimplantat ferner einen Verriegelungsstift (2) mit einer vorbestimmten Längsachse und einer vorbestimmten Form aufweist, die im Wesentlichen komplementär zu der Form der in dem Körper des Stabilisierungskeils vorgesehenen Ausnehmung ist, um mit dem Stabilisierungskeil durch Bewegung in der Ausnehmung in Richtung der Längsachse der Ausnehmung in Eingriff zu kommen, um so den sich bewegenden Riemen relativ zu dem Stabilisierungskeil durch Einklemmen des Riemenabschnitts zwischen dem Verriegelungsstift und der Innenwand der Ausnehmung zu verriegeln; und
- der langgestreckte Körper des Implantathalters rohrförmig ist, mit einem inneren Kanal (52), der sich entlang der Längsachse des langgestreckten Körpers des Implantathalters erstreckt und einen Innendurchmesser aufweist, der etwas größer ist als der größte Durchmesser des Verriegelungsstiftes (2) des Implantats, um das Einführen und Führen des Verriegelungsstiftes durch den inneren Kanal in die im Körper des Stabilisierungskeils vorgesehene Aussparung (12) in Richtung der Längsachse der Aussparung zu ermöglichen, wenn der Implantathalter an dem Stabilisierungskeil befestigt ist.

2. Chirurgie-Kit nach Anspruch 1, wobei:
- die in dem Körper des Stabilisierungskeils vorgesehene Aussparung einen mit einem Innengewinde versehenen Eintrittsbereich (11) aufweist, und
- der rohrförmige Körper des Implantathalters an seinem ersten Ende (51) ein Außengewinde aufweist, das mit dem Innengewinde des Eintrittsbereichs der Aussparung für die Schraubbefestigung des Implantathalters an dem Stabilisierungskeil (1) zusammenwirkt.

3. Chirurgie-Kit nach Anspruch 1 oder Anspruch 2, welches weiterhin umfasst:
- eine Stange (6) zum Einführen des Verriegelungsstifts (2) mit einem ersten Ende (61) und einem zweiten Ende (62), welche dazu ausgebildet ist, in dem inneren Kanal (52) des rohrförmigen Körpers des Implantathalters (5) zu gleiten, zum Einführen und Führen des Verriegelungsstifts (2) durch den inneren Kanal (52) des Implantathalters (5) bis in die in dem Körper des Stabilisierungskeils vorgesehene Aussparung (12) einzuführen und zu führen.

4. Chirurgie-Kit nach Anspruch 3, welches ferner ein Verbindungsstück (7) zur lösbaren Befestigung des Verriegelungsstiftes (2) am zweiten Ende (62) der Einführstange (6) aufweist.

5. Chirurgie-Kit nach Anspruch 4, wobei:
- der Verriegelungsstift (2) an der Rückseite (22) eine Aussparung (21) aufweist, die sich in Längsrichtung (10) des Stiftes erstreckt,
- das zweite Ende (62) der Einführstange (6) eine Aussparung mit Innengewinde aufweist, die sich in Längsrichtung der Stange (6) erstreckt, und
- das Verbindungsstück (7) ein Endstück ist, das einerseits einen ersten mit Außengewinde versehenen Endabschnitt (71) aufweist, der dazu ausgebildet ist, mit dem Innengewinde der am zweiten Ende (62) der Einführstange vorgesehenen Aussparung zusammenzuwirken, und andererseits einen zweiten konischen Endabschnitt (72) aufweist, der dazu ausgebildet ist, unter Kraftaufwand in die am hinteren Ende des Verriegelungsstifts (2) vorgesehene Aussparung einzugreifen, um die lösbare Befestigung des Verriegelungsstifts an der Einführstange zu erreichen.

6. Chirurgie-Kit nach einem der Ansprüche 1 bis 5, wobei der Implantathalter (5) an seinem zweiten Ende (53) mit einem Innengewinde an der Seite eines freien Zugangs zu dem Innenkanal (52) versehen ist, wenn der Implantathalter an dem Stabilisierungskeil befestigt ist, und wobei die Einführstange (6) an ihrem ersten Ende (61) mit einem Außengewinde zum Zusammenwirken mit dem Innengewinde versehen ist, so dass das Einführen und dann das Einschrauben der Einführstange (6) in den inneren Kanal (52) des Implantathalters (5) den konischen Stift (2) in die im Körper des Stabilisierungskeils vorgesehene Aussparung (12) in Richtung der Längsachse der genannten Aussparung treibt.

7. Chirurgie-Kit nach einem der Ansprüche 1 bis 6, wobei der Riemen eine vorbestimmte Längsrichtung und eine vorbestimmte Riemenoberfläche aufweist, und wobei der Verriegelungsstift durch Verschieben innerhalb der im Körper des Stabilisierungskeils vorgesehenen Ausnehmung derart in den Stabilisierungskeil eingreifen kann, dass die Längsachse des Verriegelungsstiftes:
- erstens dann mit der Längsachse der Aussparung zusammenfällt,
- zweitens senkrecht zur Längsachse des Riemenabschnitts innerhalb der Aussparung steht, und
- drittens parallel zur Oberfläche des ersten Riemenabschnitts innerhalb der Aussparung ist.

8. Chirurgie-Kit nach einem der Ansprüche 1 bis 6, wobei:
- der Riemen eine vorbestimmte Längsrichtung und eine vorbestimmte Riemenoberfläche aufweist;
- sowohl der erste als auch der zweite Riemenabschnitt in der im Stabilisierungskeil vorgesehenen Aufnahme verlaufen kann, so dass der Riemen in einer zur Hauptachse des Keils senkrechten Ebene wenigstens eine Schlaufe bildet, wobei wenigstens eine Rundschnur zum Eingriff in einen der Dornfortsätze von zwei zu stabilisierenden Wirbeln ausgebildet ist; und
- der Verriegelungsstift mit dem Stabilisierungskeil in Richtung der Hauptachse des Körpers des Keils zwischen dem ersten und dem zweiten Riemenabschnitt (41a, 42a) innerhalb der Aussparung in Eingriff kommen kann, so dass die Längsachse des Stiftes:
- erstens dann mit der Längsachse der Aussparung zusammenfällt;
- zweitens senkrecht zur Längsachse jedes der beiden Riemenabschnitte innerhalb der Aussparung steht; und
- drittens parallel zur Oberfläche des ersten und des zweiten Riemenabschnitts innerhalb der Aussparung ist,
und ferner derart, dass jeder aus erstem und zweitem Riemenabschnitt gegen eine Bewegung relativ zum Stabilisierungskeil verriegelt wird, indem der Riemenabschnitt zwischen dem Verriegelungsstift und jeweiligen einander zugewandten Teilen der Innenwand der Aussparung eingeklemmt wird.

9. Chirurgie-Kit nach Anspruch 8, wobei der erste und der zweite Teil des Riemens durch die im Körper des Stabilisierungskeils vorgesehene Aufnahme in entgegengesetzten Richtungen hindurch verlaufen können, so dass sie sich in der genannten Aufnahme kreuzen und der Riemen in einer zur Hauptachse des Keils senkrechten Ebene eine Schlaufe bildet, wobei zwei Rundschnüre jeweils auf beiden Seiten des Stabilisierungskeils in der genannten Ebene angeordnet und so ausgebildet sind, dass sie mit jeweils einem der Dornfortsätze zweier benachbarter zu stabilisierender Wirbel in Eingriff kommen.

10. Chirurgie-Kit nach einem der vorhergehenden Ansprüche, welches ferner eine Schraube mit dem gleichen Durchmesser umfasst wie der Durchmesser des Eintrittsbereichs der im Körper des Stabilisierungskeils vorgesehenen Aussparung, mit einem Außengewinde, das dazu ausgebildet ist, mit dem Innengewinde des Eintrittsbereichs zusammenzuwirken, und mit einem Anlagebereich (32), der dazu ausgebildet ist, an einem Kontaktbereich an der Rückseite (22) des Verriegelungsstifts (2) anzuliegen, um in der axialen Position den Eingriff des Verriegelungsstifts in der im Stabilisierungskeil vorgesehenen Aussparung (12) und somit die Verriegelung des Riemens zu verriegeln.

## Claims

1. Surgical kit comprising:
- an intervertebral implant with:
- a stabilizing wedge (1) adapted to stabilize at least two adjacent vertebrae by interposition between spinous processes of the vertebrae, having a substantially parallelepipedal body which has a defined main axis (10) and in which is provided a recess (12) having a defined longitudinal axis parallel to the main axis of the body of the stabilizing wedge; and
- at least one strap (4) forming a flexible link for fixing the stabilizing wedge to the spinous processes of the vertebrae to be stabilized, said strap having first and second portions (41a, 42a) which each comprising one of the opposite ends (41, 42) of said strap; and also
- an implant holder (5) having an elongate body along a defined longitudinal axis and with a first end (51) and a second end (53), and having fixing means which are provided at the first end (51) and which are adapted to cooperate with associated means provided at the body of the stabilizing wedge of the implant for fixing the implant holder to the body of the stabilizing wedge in such a way that the elongate body of said implant holder is in parallax with said recess (12) ;
in which:
- the recess provided in the body of the stabilizing wedge comprises an inner wall extending parallel to the longitudinal axis of the recess and has a defined shape;
- at least the first strap portion can pass through the recess perpendicularly with respect to the longitudinal axis of said recess;
- the intervertebral implant further comprises a blocking pin (2) having a defined longitudinal axis and a defined shape substantially complementing the shape of the recess provided in the body of the stabilizing wedge, in order to come into engagement with said stabilizing wedge by movement in said recess in the direction of the longitudinal axis of said recess, so as to immobilize the strap with respect to the stabilizing wedge by clamping the strap portion between the blocking pin and the inner wall of the recess; and
- the elongate body of the implant holder is tubular with an internal channel (52) extending along the longitudinal axis of the elongate body of the implant holder and having an internal diameter slightly larger than the largest diameter of the blocking pin (2) of the implant, in order to allow said blocking pin (2) to be inserted and guided through said internal channel, as far as the recess (12) provided in the body of the stabilizing wedge, in the direction of the longitudinal axis of said recess, when the implant holder is fixed to the stabilizing wedge.

2. Surgical kit according to Claim 1, in which:
- the recess provided in the body of the stabilizing wedge has an internally threaded inlet zone (11), and
- the tubular body of the implant holder has, at its first end (51), a thread adapted to cooperate with the internal thread of the inlet zone of the recess in order to fix the implant holder to the stabilizing wedge (1) by screwing.

3. Surgical kit according to Claim 1 or Claim 2, further comprising:
- an insertion rod (6) for the blocking pin (2), having a first end (61) and a second end (62) and being adapted to slide in the internal channel (52) of the tubular body of the implant holder (5) for inserting and guiding the blocking pin (2) through the internal channel (52) of the implant holder (5) as far as the recess (12) provided in the body of the stabilizing wedge.

4. Surgical kit according to Claim 3, further comprising a connecting piece (7) for fixing the blocking pin (2) detachably to the second end (62) of the insertion rod (6).

5. Surgical kit according to Claim 4, in which:
- the blocking pin (2) has, at the rear (22), a recess (21) extending in the longitudinal direction (10) of said pin,
- the second end (62) of the insertion rod (6) comprises an internally threaded recess extending in the longitudinal direction of said rod (6), and
- the connecting piece (7) is a ferrule having, on the one hand, a first threaded end part (71) adapted to cooperate with the internal thread of the recess provided at the second end (62) of the insertion rod, and, on the other hand, a second, conical end part (72) adapted to be engaged by force into the recess provided at the rear of the blocking pin (2) in order to achieve the detachable fixing of said blocking pin to said insertion rod.

6. Surgical kit according to one of Claims 1 to 5, in which the implant holder (5) is provided with an internal thread at its second end (53), at a free inlet of the internal channel (52) when the implant holder is fixed to the stabilizing wedge, and in which the insertion rod (6) is provided, at its first end (61), with a thread in order to cooperate with said internal thread in such a way that the insertion and then the screwing of the insertion rod (6) into the internal channel (52) of the implant holder (5) drives the conical pin (2) inside the recess (12) provided in the body of the stabilizing wedge in the direction of the longitudinal axis of said recess.

7. Surgical kit according to any one of Claims 1 to 6, in which the strap has a defined longitudinal direction and a defined strap surface, and in which the blocking pin can come into engagement with the stabilizing wedge by movement inside the recess provided in the body of the stabilizing wedge, in such a way that the longitudinal axis of the blocking pin:
- firstly coincides with the longitudinal axis of the recess;
- secondly is perpendicular to the longitudinal axis of the strap portion inside the recess; and
- thirdly is parallel to the surface of the first strap portion inside the recess.

8. Surgical kit according to any one of Claims 1 to 6, in which:
- the strap has a defined longitudinal direction and a defined strap surface;
- each of the first and second strap portions can pass through the seat provided in the stabilizing wedge, in order for the strap to form at least one loop in a plane perpendicular to the main axis of the wedge, with at least one cord adapted to come into engagement with one of the spinous processes of two vertebrae to be stabilized; and
- the blocking pin can come into engagement with the stabilizing wedge in the direction of the main axis of the body of said wedge, between each of the first and second strap portions (41a, 42a) inside the recess, in such a way that the longitudinal axis of the pin:
- firstly coincides with the longitudinal axis of the recess;
- secondly is perpendicular to the longitudinal axis of each of the two portions of the strap inside the recess; and
- thirdly is parallel to the surface of each of the first and second strap portions inside the recess,
and moreover in such a way that each of the first and second strap portions is immobilized with respect to the stabilizing wedge by said strap portion being clamped between the blocking pin and respective portions of the inner wall of the recess that face each other.

9. Surgical kit according to Claim 8, in which the first and second strap portions can pass in opposite directions through the seat provided in the body of the stabilizing wedge, so as to intersect in said seat, and in order for the strap to form a loop in a plane perpendicular to the main axis of the wedge, with two cords which are located respectively on either side of the stabilizing wedge in said plane and are adapted to come into engagement each with a respective one of the spinous processes of two adjacent vertebrae to be stabilized.

10. Surgical kit according to any one of the preceding claims, further comprising a screw of the same diameter as the diameter of the inlet zone of the recess provided in the body of the stabilizing wedge, with a thread adapted to cooperate with the internal thread of said inlet zone, and with a bearing zone (32) adapted to bear against a contact zone at the rear (22) of the blocking pin (2) in order to axially lock the engagement of the blocking pin in the recess (12) provided in the stabilizing wedge and thus the blocking of the strap.
